Europäisches Patentamt

European Patent Office

Office européen des brevets

(11) Veröffentlichungsnummer : **0 012 282**
**B1**

(12) **EUROPÄISCHE PATENTSCHRIFT**

(45) Veröffentlichungstag der Patentschrift :
**20.04.83**

(21) Anmeldenummer : **79104776.4**

(22) Anmeldetag : **29.11.79**

(51) Int. Cl.³ : **A 61 B  5/10**, A 61 C  19/04,
**G 01 B  7/00**, G 01 B  7/31

(54) **Gerät zur Messung und Registrierung des Ortes, der Lage und/oder der Orts- bzw. Lageänderung eines starren Körpers im Raum.**

(30) Priorität : **06.12.78 DE 2852764**

(43) Veröffentlichungstag der Anmeldung :
**25.06.80 Patentblatt 80/13**

(45) Bekanntmachung des Hinweises auf die Patenterteilung : **20.04.83 Patentblatt 83/16**

(84) Benannte Vertragsstaaten :
**FR GB IT**

(56) Entgegenhaltungen :
**FR A 2 386 807**
**US A 3 971 983**

(73) Patentinhaber : **SIEMENS AKTIENGESELLSCHAFT**
**Berlin und München Wittelsbacherplatz 2**
**D-8000 München 2 (DE)**

(72) Erfinder : **Nickel, Bernd, Dipl.-Ing.**
**Goldregenstrasse 2**
**D-6143 Lorsch (DE)**
Erfinder : **Schorr, Wolfgang**
**Am Teufelsbach 8**
**D-6147 Lautertal 3 (DE)**

Gerät zur Messung und Registrierung des Ortes, der Lage und/oder der Orts- bzw. Lageänderung eines starren Körpers im Raum

Die Erfindung bezieht sich auf ein Gerät zur Messung und Registrierung des Ortes, der Lage und/oder der Orts- bzw. Lageänderung eines starren Körpers im Raum mit einem am Körper befestigbaren Magnetfelderzeuger, der ein definiertes unregelmäßiges Feld erzeugt, mit mehreren im Abstand vom Magnetfelderzeuger anzuordnenden, vom Körper unabhängigen Magnetflußaufnehmern, welche feldflußabhängige Sensorelemente zur Erfassung des Feldflusses bzw. einer Feldflußänderung während einer Messung enthalten, sowie mit einer mit den Magnetflußaufnehmern verbundenen elektronischen Einrichtung zur Gewinnung und Auswertung von bei einem Feldfluß bzw. einer Feldflußänderung entstehenden elektrischen Signalen.

Geräte dieser Art sind in der DE-A1-27 15 106 und EP-A1-00 04 888 beschrieben. Bei beiden Geräten dient als Felderzeuger ein V-förmiger Permanentmagnet, der am Körper, z. B. am Unterkiefer eines Patienten, befestigt wird. Zur Erfassung des Feldflusses bzw. einer Feldflußänderung sind bei dem Gerät nach DE-A1-27 15 106 drei plattenförmige, bei dem Gerät nach EP-A1-00 04 888 sechs stabförmige Magnetflußaufnehmer vorgesehen. Letztere sind paarweise in drei senkrecht zueinander stehenden Ebenen angeordnet ; wodurch, wie bei dem Gerät nach DE-A1-27 15 106 drei senkrecht zueinander stehende Aufnahmeflächen gebildet werden. Das gesamte Aufnehmersystem ist bei beiden Geräten seitlich versetzt am Patienten angeordnet. Wegen der asymmetrischen Anordnung des Systems ergeben sich nichtlineare Signale, die, um überhaupt auswertbar zu sein, auf elektronischem Wege mit einem entsprechenden Schaltungsaufwand linearisiert werden müssen. Die Bedienung der Geräte ist außerdem im Hinblick auf die Justierung der Magnetfeldaufnehmer und bezüglich des Nullabgleichs der Elektronik für den Benutzer zu kompliziert und zeitaufwendig. Der Abgleich des Linearisierungsverstärkers ist außerdem problematisch, weil sich eine Ungenauigkeit, z. B. hervorgerufen durch eine Temperaturdrift, wegen der nichtlinearen Kennlinie der zur Anwendung kommenden Sensorelemente (in der Regel Hallgeneratoren), die die Aufnehmer beinhalten, auf die Empfindlichkeit und damit auf die Reproduzierbarkeit der gewonnenen Signale auswirkt.

Aufgabe der Erfindung ist es, ein demgegenüber verbessertes Gerät der genannten Art anzugeben, das insbesondere einen geringeren Schaltungsaufwand zur Signalauswertung erfordert und das von der Bedienung her einfacher ist, so daß auch ein Nichtfachmann das Justieren der Aufnehmer leicht bewerkstelligen kann.

Zur Lösung der gestellten Aufgabe wird gemäß der Erfindung vorgeschlagen, daß die Magnetflußaufnehmer in zwei Gruppen mit jeweils gleicher Anzahl auf einem ortsfesten Träger einander gegenüberstehend angeordnet sind und parallel zueinander und senkrecht zur wirksamen Fläche der Sensorelemente verlaufende, die Feldlinien konzentrierende stabförmige Antennen enthalten, deren dem Felderzeuger zugewandte Enden die Eckpunkte eines Quaders bilden.

Mit der gemäß der Erfindung vorgeschlagenen symmetrischen Anordnung der Magnetflußaufnehmer läßt sich ein einfacherer Schaltungsaufwand zur Auswertung der von den Aufnehmern gewonnenen Signale erzielen. Nachdem die Magnetflußaufnehmer zu beiden Seiten des Patientenkopfes angeordnet werden können, bleibt der Zugang zum Patientenmund weitgehend frei.

Vorteilhafte Ausgestaltungen und Weiterbildungen der Erfindung sind in den Unteransprüchen enthalten. Ein Ausführungsbeispiel der Erfindung wird nachstehend anhand der Zeichnung näher erläutert :

Es zeigen :

Figur 1  das erfindungsgemäße Gerät in einer schaubildlichen Darstellung,

Figur 2  einen Teil des Aufnehmersystems in schaubildlicher Darstellung,

Figur 3  einen Magnetflußaufnehmer des in Fig. 2 gezeigten Aufnehmersystems in einer Explosionsdarstellung,

Figur 4  eine perspektivische Darstellung des durch die Eckpunkte der einzelnen Aufnehmer gebildeten Quaders,

Figuren 5 und 6   Blockschaltbilder zur Signalauswertung.

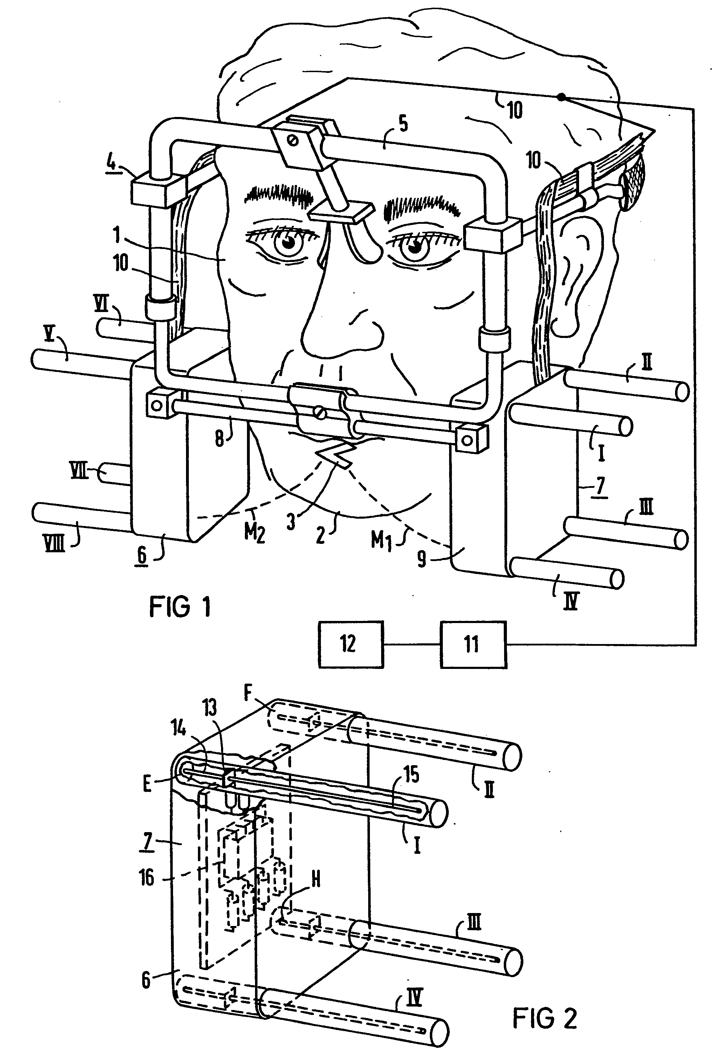

Die Fig. 1 zeigt in einer schaubildlichen Darstellung das erfindungsgemäße Gerät in einer besonders vorteilhaften Anwendung, nämlich in der Zahnmedizin, zur Bestimmung des Ortes, der Lage und/oder einer Orts- bzw. Lageänderung eines Punktes des Unterkiefers eines Patienten. In der Figur ist mit 1 der Kopf eines Patienten und mit 2 dessen Unterkiefer bezeichnet. Mit 3 ist ein als Felderzeuger dienender Permanentmagnet bezeichnet, der intraoral an einer beliebigen Stelle des Unterkiefers durch geeignete Haft- oder Klebemittel (z. B. Abdruckmasse) befestigt wird. Der Magnetfelderzeuger 3 besteht aus zwei gleichdimensionierten Stabmagneten, wie sie in der deutschen Patentanmeldung P 27 15 106 näher beschrieben sind. Der Öffnungswinkel der beiden Stabmagnete beträgt etwa 90°. Die Stabmagnete sind relativ klein ; sie haben etwa eine Länge von 3 mm und einen Querschnitt von etwa 1 mm im Quadrat. Der Magnetfelderzeuger 3 erzeugt zwei in der Figur gestrichelt angedeutete, unregelmäßige, nicht rotationssymmetrische Magnetfelder $M_1$ und $M_2$.

Extraoral des Patientenmundes befindet sich eine Magnetflußaufnehmeranordnung 4, bestehend im wesentlichen aus einem am Patientenkopf 1 gehalterten Gestell 5 und einem Aufnehmersystem mit einem links und rechts vom Unterkiefer befindlichen Aufnehmerblock 6 und 7. Das Gestell 5 ist in bekannter Weise als kombiniertes Brillen- oder Kopfgestell ausgebildet und enthält mehrere, nicht näher bezeichnete Gelenke zur Anpassung an die unterschiedlichen Kopfkonstellationen eines Patienten. Die beiden Aufnehmerblöcke 6, 7 sind durch eine mit dem Gestell 5 verbundene Stange 8 starr miteinander verbunden.

Jeder der Aufnehmerblöcke 6, 7 enthält vier Magnetflußaufnehmer I bis VIII, die in einem Kunststoffgehäuse 9 gehaltert sind, und zwar so, daß sie jeweils parallel zueinander liegen. Die von den Magnetflußaufnehmern I bis VIII aufgenommenen Signale werden über Leitungen 10 einer elektronischen Auswerteinrichtung 11 und von dort aus weiter einer geeigneten Indikatoreinrichtung 12 zugeführt.

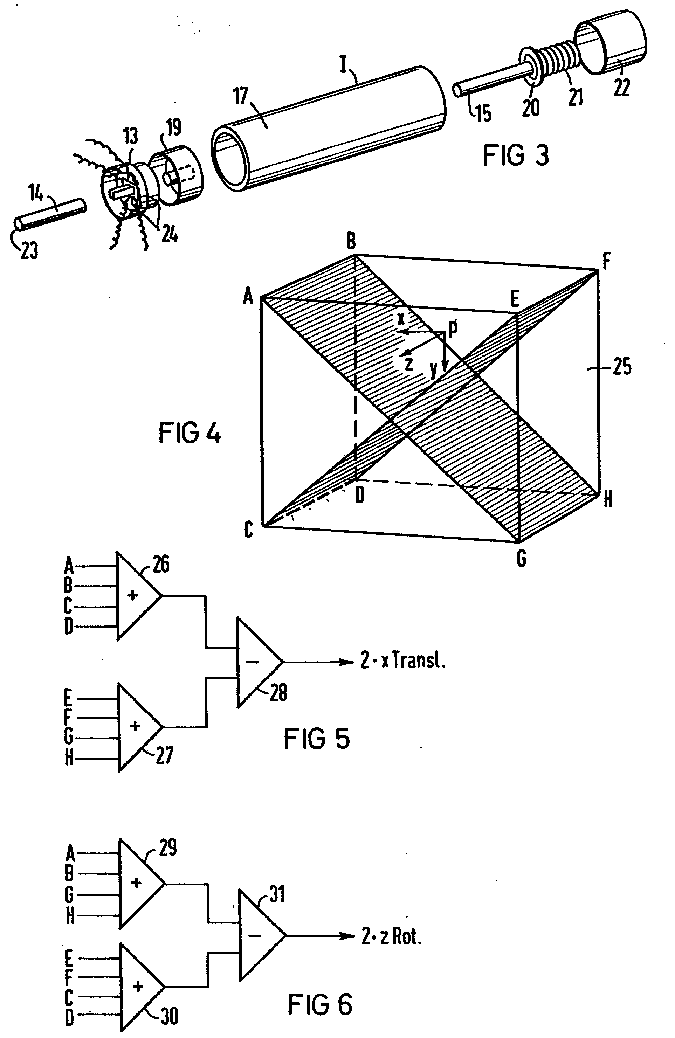

Die Fig. 2 läßt anhand des Magnetflußaufnehmerblockes 7 die Anordnung der Magnetflußaufnehmer I bis VIII erkennen. Jeder Magnetflußaufnehmer enthält einen als Sensorelement dienenden plättchenförmigen Hallgenerator 13, an dessen wirksame Fläche (in Fig. 3 mit 24 bezeichnet) beidseitig verschieden lange Antennenstäbe 14, 15 aus Mu-Metall anliegen. Die von den Magnetflußaufnehmern I bis VIII bzw. von den Hallgeneratoren 13 aufgenommenen Signale werden über den im Gehäuse 9 angeordneten Vorverstärker 16 verstärkt der Elektronikeinheit 11 zugeführt. Durch die Anordnung eines Vorverstärkers in den beiden Gehäusen 9 läßt sich eine Reduzierung der Anzahl der Leitungen 10 zur Elektronikeinheit 11 erzielen.

Die Fig. 3 zeigt in einer Explosionsdarstellung anhand des Magnetflußaufnehmers I den Aufbau der stabförmigen Magnetflußaufnehmer. Der Aufnehmer enthält eine Hülse 17 aus nicht ferromagnetischem Material, vorzugsweise aus Kunststoff, in die ein aus zwei Teilen 18, 19 bestehendes Tragteil für den Hallgenerator 13 eingeschoben wird. Die beiden Tragteile 18, 19 sind mit entsprechenden Bohrungen zur Führung einerseits des kürzeren Antennenstabes 14 und andererseits des längeren Antennenstabes 15 versehen. Die Tragteile 18 und 19 sind im zusammengefügten Zustand in der Hülse 17 längsbeweglich geführt. Am Antennenstab 15 ist eine Scheibe 20 befestigt, an der sich eine Druckfeder 21 abstützt, die mit ihrem anderen Ende an einem im montierten Zustand in der Hülse 17 fest angeordneten Verschlußstopfen 22 stirnseitig anliegt. Die Anordnung ermöglicht, daß das Aufnehmersystem, bestehend aus den beiden Antennen 14 und 15 und dem in den Teilen 18 und 19 gehalterten Hallgenerator 13 zum Patientenkopf hin gedrückt wird. Das dem Gehäuse 9 zugewandte Ende 23 des Antennenstabes 14 liegt so an einer dem Patientenkopf unmittelbar benachbarten Fläche des Gehäuses 9 an. Der sich dabei ergebende Festpunkt ist in Fig. 2 für den Aufnehmer I des Aufnehmerblockes 7 mit E bezeichnet. Für die übrigen Aufnehmer II bis VIII ergeben sich analog die Festpunkte A bis D und F bis G. Infolge der federnden Anordnung der Teile werden die Antennenstäbe 14 und 15 gegen die wirksamen Flächen 24 der plättchenförmigen Hallgeneratoren gedrückt, wodurch die Antennenempfindlichkeit erhöht wird.

Durch die symmetrische Anordnung der Hallgeneratoren 13 in der aufgezeigten Weise bilden diese bzw. die Festpunkte A bis H, die durch das Anliegen der Enden 23 der den Hallgeneratoren 13 zugeordneten Antennenstäbe 14 am Gehäuse 9 gebildet werden, den in Fig. 4 mit 25 bezeichneten Quader. Der Magnetfelderzeuger 3 ist, wie aus Fig. 1 zu ersehen ist, innerhalb des durch den Quader 25 gebildeten Raumes angeordnet. Ausgehend von einem in diesem Raum angenommenen Punkt P, in dem sich der Magnetfelderzeuger 3 befindet, werden durch die symmetrische Anordnung der Magnetfeldaufnehmer an den für die Antennenwirkung repräsentativen Eckpunkten A bis H bezüglich einer Translationsbewegung des Unterkiefers bzw. des Felderzeugers in den drei Ebenen x, y und z Signale entsprechend folgender Beziehungen gewonnen :

$$A + B + C + D \text{ prop. } x$$
$$C + D + G + H \text{ prop. } y$$
$$A + E + C + G \text{ prop. } z$$

Bei einer Bewegung in die Gegenrichtung ergibt sich analog :

$$E + F + G + H \text{ prop. } -x$$
$$A + B + E + F \text{ prop. } -y$$
$$B + F + D + H \text{ prop. } -z$$

Durch Verknüpfung der Signale entsprechend dem Blockschaltbild nach Fig. 5 durch jeweils einen Additionsverstärker 26, 27 und einen Differenzverstärker 28 erhält man ein entsprechend auswertbares Nutzsignal 2x. Für die Signale y und z gilt analoges.

Für die Aufnahme einer Rotationsbewegung werden die Signale von jeweils zwei diagonal einander gegenüberliegenden Eckpunktpaaren mit den komplementären Eckpunktpaaren differentiell miteinander verknüpft. In Fig. 4 sind die sich hieraus ergebenden Diagonalflächen für die Signalverarbeitung einer Rotationsbewegung um die z-Achse schraffiert eingezeichnet. Für die Signalverarbeitung gilt :

$$A + B + G + H \text{ prop. Rot. } z$$
$$A + E + D + H \text{ prop. Rot. } x$$
$$B + D + E + G \text{ prop. Rot. } y$$

Für die Gegenrichtung gilt entsprechend :

$$E + F + C + D \text{ prop.} - \text{Rot. z}$$
$$B + F + C + G \text{ prop.} - \text{Rot. x}$$
$$A + C + F + H \text{ prop.} - \text{Rot. y}$$

Die Signale werden auch hier über Additionsverstärker 29 und 30 und einem Differenzverstärker 31 entsprechend der nachstehenden Beziehung verarbeitet :

$$(A + E + D + H) - (B + F + C + G) \text{ prop. } 2 \cdot \text{Rot. x}$$
$$(B + D + E + G) - (A + C + F + H) \text{ prop. } 2 \cdot \text{Rot. y}$$
$$(A + B + G + H) - (E + F + C + D) \text{ prop. } 2 \cdot \text{Rot. z}$$

Entsprechend der Signalverarbeitung nach den Fig. 5 und 6 können in den drei Ebenen x, y und z auf einfache Weise Direktsignale für eine Translations- und eine Rotationsbewegung gewonnen werden. Dadurch, daß jeder Eckpunkt für mehrere Flächen ausgenutzt wird, z. B. der Eckpunkt A sowohl für die Fläche A, B, C, D als auch für die Fläche A, E, G, C und A, B, E, F, ergibt sich ein einfacher Aufbau des gesamten Aufnehmersystems. Durch das erläuterte Prinzip der Differenzbildung der Signale der jeweils komplementären Ebene läßt sich außerdem ein weit größerer Linearitätsbereich schaffen als bei dem bisherigen System, wo aufgrund der Charakteristik des Systems Magnet/Antenne an sich ein begrenzter Linearitätsbereich gegeben und dadurch zusätzliche Linearisierungsverstärker notwendig waren.

**Ansprüche**

1. Gerät zur Messung und Registrierung des Ortes, der Lage und/oder der Orts- bzw. Lageänderung eines starren Körpers im Raum mit einem am Körper befestigbaren Magnetfelderzeuger (3), der ein definiertes unregelmäßiges Feld erzeugt, mit mehreren im Abstand vom Magnetfelderzeuger (3) anzuordnenden, vom Körper unabhängigen Magnetflußaufnehmern (I-VIII), welche feldflußabhängige Sensorelemente (13) zur Erfassung des Feldflusses bzw. einer Feldflußänderung während einer Messung enthalten, sowie mit einer mit den Magnetflußaufnehmern (I-VIII) verbundenen elektronischen Einrichtung (II) zur Gewinnung und Auswertung von bei einem Feldfluß bzw. einer Feldflußänderung entstehenden elektrischen Signalen, dadurch gekennzeichnet, daß die Magnetflußaufnehmer (I bis VIII) in zwei Gruppen (I-IV ; V-VIII) mit jeweils gleicher Anzahl auf einem ortsfesten Träger (5-8) einander gegenüberstehend angeordnet sind und parallel zueinander und senkrecht zur wirksamen Fläche (24) der Sensorelemente (13) verlaufende, die Feldlinien konzentrierende stabförmige Antennen (14, 15) enthalten, deren dem Felderzeuger (3) zugewandte Enden (23) die Eckpunkte (A-H) eines Quaders (25) bilden.

2. Gerät nach Anspruch 1, dadurch gekennzeichnet, daß die Antennen (14, 15) mittels Federelemente (21) an die wirksamen Flächen (24) der Sensorelemente (13) angedrückt werden.

3. Gerät nach Anspruch 1 oder 2, dadurch gekennzeichnet, daß beidseitig der Sensorelemente (13) ein Antennenstab (14, 15) vorhanden ist.

4. Gerät nach einem der Ansprüche 1 bis 3, dadurch gekennzeichnet, daß die Sensorelemente (13) in Aufnahmeteilen (18, 19) aus nicht ferromagnetischem Material, vorzugsweise aus Kunststoff, angeordnet sind, welche beidseitig eine Führung für die Antennenstäbe (14, 15) enthalten, und die Aufnahmeteile (18, 19) in einer Hülse (17) längsbeweglich geführt sind und mittels eines Federelementes (21) gegen einen einen Eckpunkt des Quaders (25) bildenden Bezugspunkt (E) gedrückt wird.

5. Gerät nach einem der Ansprüche 1 bis 4, dadurch gekennzeichnet, daß jeweils vier Aufnehmer (I bis IV) in einem Gehäuse (9) zur Aufnahme eines elektrischen Vorverstärkers (16) angeordnet sind.

6. Gerät nach einem der Ansprüche 1 bis 5, dadurch gekennzeichnet, daß zur Erfassung der Translationsbewegung die von den Sensorelementen (13) jeweils einer Fläche (A, B, C, D) des Quaders (25) gebildeten Signale einem Additionsverstärker (26) zugeführt werden.

7. Gerät nach einem der Ansprüche 1 bis 6, dadurch gekennzeichnet, daß zur Ausschaltung von äußeren Störfeldern die Signale von jeweils zwei parallelen Flächen (A, B, C, D und E, F, G, H) Additionsverstärkern (26, 27) zugeführt werden, die daraus zwei Flächensignale bilden, die über einen Differenzverstärker (28) verknüpft das doppelte Nutzsignal (2 · x-Transl.) und ein zu Null kompensiertes Störsignal liefern.

8. Gerät nach einem der Ansprüche 1 bis 7, dadurch gekennzeichnet, daß zur Erfassung der Rotationsbewegung die Signale von jeweils zwei diagonal einander gegenüberliegenden Eckpunktpaaren (A, B, G, H) der Magnetfeldaufnehmer (I bis VIII) einem Additionsverstärker (29) zugeführt werden.

9. Gerät nach Anspruch 8, dadurch gekennzeichnet, daß die Signale von jeweils zwei komplementären Diagonalflächen (A, B, G, H und C, D, E, F) Additionsverstärkern (29 und 30) zugeführt werden, die daraus zwei Flächensignale bilden, die über einen Differenzverstärker (31) verknüpft das doppelte Nutzsignal (2 · z-Rot.) und ein zu Null kompensiertes Störsignal liefern.

**Claims**

1. Device for measuring and registrating the location, the attitude and/or the change of location or attitude, as the case may be, of a rigid body in space comprising a magnetic field generator (3) which can be secured to the body and which produces a defined, irregular field, with a plurality of magnetic flux receivers (I-VIII) which are independent of the body and which are to be spaced from the magnetic field generator (3) and which contain field-flux-dependent sensor elements (13) for detecting the field flux or a change of field flux, as the case may be, during a measurement, and with an electronic device (II), which is connected to the magnetic flux receivers (I-VIII), for obtaining and analysing electric signals which occur in relation to a field flux or a change of field flux, as the case may be, characterised in that the magnetic flux receivers (I to VIII) are arranged in two groups (I-IV ; V-VII), each of the same number, on a stationary carrier (5-8) so as to be opposite one another and parallel to one another and perpendicular to the effective surface (24) of the sensor elements (13) which contain rod-shaped antennae (14, 15) which concentrate field lines and whose ends (23) facing the field generator (3) form the corner points (A-H) of a parallelepiped (25).

2. Device as claimed in claim 1, characterised in that the antennae (14, 15) are pressed against the effective surfaces (24) of the sensor elements (13) by means of spring elements (21).

3. Device as claimed in claim 1 or 2, characterised in that an antenna rod (14, 15) is arranged on both sides of the sensor elements (13).

4. Device as claimed in one of claims 1 to 3, characterised in that the sensor elements (13) are arranged in accommodating members (18, 19) which comprise ferromagnetic material, preferably a synthetic material, and comprise a guide for the antenna rods (14, 15) on both sides, and that the accommodating members (18, 19) are guided in a sleeve (17) so as to be longitudinally movable and are pressed against a reference point (E) forming a corner point of the parallelepiped (25).

5. Device as claimed in one of claims 1 to 4, characterised in that in each case four receivers (I to IV) are arranged in a housing (9) for accommodating an electric pre-amplifier (16).

6. Device as claimed in one of claims 1 to 5, characterised in that in order to detect translational motion the signals, which are formed by the sensor elements (13) of one surface (A, B, C, D) of the parallelepiped (25) are supplied to an addition amplifier (26).

7. Device as claimed in one of claims 1 to 6, characterised in that in order to eliminate exterior noise fields the signals of two parallel surfaces (A, B, C, D and E, F, G, H) are fed to addition amplifiers (26, 27) which form two surface signals therefrom which, linked by means of a difference amplifier (28), supply the doubled useful signal (2 · x-transl.) and an interfering signal compensated to zero.

8. Device as claimed in one of claims 1 to 7, characterised in that in order to detect rotational motion the signals of two pairs of corner points (A, B, G, H), which are located opposite one another so as to be diagonal, of the magnetic flux receivers (I to VIII) are fed to an addition amplifier (29).

9. Device as claimed in claim 8, characterised in that the signals of two complementary diagonal surfaces (A, B, C, H and C, D, E, F) are fed to addition amplifiers (29, 30) which form two surface signals therefrom which, linked by means of a difference amplifier (31), supply the doubled useful signal (2 · z-rot.) and an interfering signal which is compensated to zero.

**Revendications**

1. Appareil pour mesurer et enregistrer l'emplacement, la position et/ou la modification d'emplacement ou de position d'un corps rigide dans l'espace, comprenant un générateur de champ magnétique (3) pouvant être fixé au corps et donnant un champ défini irrégulier, plusieurs capteurs de flux magnétique (I à VIII) indépendants du corps, à disposer à distance du générateur de champ magnétique (3) et contenant des éléments de détecteur (13) dépendants du flux du champ pour la détection du flux du champ ou d'une variation de flux du champ pendant une mesure, ainsi qu'un dispositif (II) électronique relié aux capteurs de flux magnétique (I à VIII) pour l'obtention et l'exploitation de signaux électriques créés lors d'un flux du champ ou lors d'une variation du flux du champ, caractérisé en ce que les capteurs de flux magnétique (I à VIII) sont disposés en opposition mutuelle en deux groupes (I à IV ; V à VIII) de même nombre sur un support (5 à 8) fixe et contiennent des antennes (14, 15) en forme de barrettes, qui s'étendent parallèlement les unes aux autres et perpendiculairement aux surfaces efficaces (24) des éléments de détecteur (13), qui concentrent les lignes de champ et dont les extrémités (23) se trouvant du côté du générateur de champ (3) forment les sommets (A à H) d'un parallélépipède (25).

2. Appareil suivant la revendication 1, caractérisé en ce que les antennes (14, 15) sont pressées sur les surfaces efficaces (24) des éléments de détecteur (13) au moyen d'éléments à ressort (21).

3. Appareil suivant la revendication 1 ou 2, caractérisé en ce qu'une barrette formant antenne (14, 15) se trouve des deux côtés des éléments de détecteur (13).

4. Appareil suivant l'une des revendications 1 à 3, caractérisé en ce que les éléments de détecteur (13) sont disposés dans des parties de réception (18, 19) en un matériau qui n'est pas ferromagnétique, de préférence en matière plastique, qui comprennent des deux côtés un guidage pour les barrettes formant antennes (14, 15), et les parties de réception (18, 19) sont guidées avec possibilité de déplacement longitudinal dans une douille (17) et sont pressées au moyen d'un élément à ressort (21) sur un point de référence (E) formant l'un des sommets du parallélépipède (25).

5. Appareil suivant l'une des revendications 1 à 4, caractérisé en ce que respectivement quatre

**0 012 282**

capteurs (I à IV) sont disposés dans un boîtier (9) de réception d'un préamplificateur électrique (16).

6. Appareil suivant l'une des revendications 1 à 5, caractérisé en ce que, pour la détection du déplacement en translation, les signaux formés par les éléments de détecteur (13) de chaque face (A, B, C, D) du parallélépipède (25) sont envoyés à un amplificateur additionneur (26).

7. Appareil suivant l'une des revendications 1 à 6, caractérisé en ce que, pour la mise hors circuit de champs parasites extérieurs, les signaux de deux faces parallèles (A, B, C, D et E, F, G, H) sont envoyés à des amplificateurs additionneurs (26, 27) qui forment, à partir de ceux-ci, deux signaux de surface qui, appliqués à un amplificateur différentiel (28), fournissent le double du signal utile (2 · x-transl.) et un signal parasite compensé jusqu'à ce qu'il soit égal à zéro.

8. Appareil suivant l'une des revendications 1 à 7, caractérisé en ce que, pour la détection du déplacement en rotation, les signaux de deux paires de sommets diagonalement opposés (A, B, G, H) des capteurs de champ magnétique (I à VIII) sont envoyés à un amplificateur additionneur (29).

9. Appareil suivant la revendication 8, caractérisé en ce que les signaux de deux faces diagonales complémentaires (A, B, G, H et C, D, E, F) sont envoyés à des amplificateurs additionneurs (29 et 30) qui forment, à partir de ces signaux, deux signaux de surface qui, appliqués à un amplificateur différentiel (31), fournissent le double du signal utile (2 · z-Rot.) et un signal parasite compensé jusqu'à ce qu'il soit égal à zéro.

6

FIG 1

FIG 2

FIG 3

FIG 4

FIG 5

FIG 6

2· xTransl.

2· zRot.